# EUROPEAN PATENT SPECIFICATION

(11) **EP 3 882 629 B1**
(45) Date of publication and mention of the grant of the patent: **30.11.2022**
(21) Application number: 21020152.1
(22) Date of filing: 16.03.2021
(51) Int. Cl.: G01N 33/50, G01N 30/70

(54) **MONITORING MYCOTOXINS AND ITS METABOLITES IN THE BLOOD OF PIGS OR BROILER CHICKENS**
ÜBERWACHUNG VON MYKOTOXINEN UND DEREN METABOLITEN IM BLUT VON SCHWEINEN ODER MASTHÄHNCHEN
SURVEILLANCE DES MYCOTOXINES ET DE LEURS MÉTABOLITES DANS LE SANG DES PORCS OU DES POULETS DE CHAIR

(30) Priority: 17.03.2020 BE 202000030
(43) Date of publication of application: 22.09.2021
(73) Proprietor: Innov Ad N.V., 2910 Essen (BE); Universiteit Gent, 9000 Gent (BE)
(72) Inventor: Lauwers, Marianne, 9820 Merelbeke (BE); Croubels, Siska, 9820 Merelbeke (BE); Devreese, Mathias, 9820 Merelbeke (BE); De Baere, Siegrid, 9820 Merelbeke (BE); Sevastiyanova, Milena, B-2910 Essen (BE); Gougoulias, Christos, B-2910 Essen (BE); Letor, Ben, B-2910 Essen (BE)
(74) Representative: Theunis, Patrick

(56) References cited:
- MARIANNE LAUWERS ET AL: "Assessment of Dried Blood Spots for Multi-Mycotoxin Biomarker Analysis in Pigs and Broiler Chickens", TOXINS, vol. 11, no. 9, 2019, page 541, XP055744557,
- MARIANNE LAUWERS ET AL: "Biomarkers for Exposure as A Tool for Efficacy Testing of A Mycotoxin Detoxifier in Broiler Chickens and Pigs", TOXINS, vol. 11, no. 4, 2019, page 187, XP055744558,
- S. DE BAERE ET AL: "Development of a liquid-chromatography tandem mass spectrometry and ultra-high-performance liquid chromatography high-resolution mass spectrometry method for the quantitative determination of zearalenone and its major metabolites in chicken and pig plasma", ANALYTICA CHIMICA ACTA, vol. 756, 2012, pages 37-48, XP055744811,
- ULRIKE BREZINA ET AL: "Diagnosis of intoxications of piglets fed with Fusarium toxin-contaminated maize by the analysis of mycotoxin residues in serum, liquor and urine with LC-MS/MS", ARCHIVES OF ANIMAL NUTRITION, vol. 68, no. 6, 2014, pages 425-447, XP055744829,
- DE RUYCK KARL ET AL: "Mycotoxin exposure assessments in a multi-center European validation study by 24-hour dietary recall and biological fluid sampling", ENVIRONMENT INTERNATIONAL, vol. 137, 5 February 2020 (2020-02-05), XP086074506,

## Description

### Field of the Invention

The present invention relates to a novel and inventive method for enhancing productivity in the agricultural business. More in particular, the present invention relates to a novel and inventive method for biomonitoring mycotoxins and their phase I and phase II metabolites in an easy and user-friendly manner via accessible animal matrices, and more specifically in the blood of broiler chickens or pigs.

### Background prior art of the invention

The need for increased protein consumption via meat production in a sustainable manner globally, is directly linked with the need for reduction of the overall production cost. This certainly remains the primary objective within the pig and broiler chicken industry with the feed cost having the biggest impact. The high and unpredictable variability of raw material costs (cereals, proteins and fats) and the ever growing and complex load of toxins contamination continue to negatively impact productivity.

Several stress factors such as handling, sudden environmental changes, heat, feed programs & diet composition changes, vaccination, disease challenges, infections and impaired immune response are affecting animal health and productivity. Modern farming animals possess a limited natural resistance and immunity against such stresses leading often to oxidative stress, when the animal is no longer capable of detoxifying timely the reactive oxygen species at cell level. The impact of such stress factors is worsened and amplified in the presence of mycotoxins.
- Mycotoxins decrease the function of key metabolic organs such as the liver and kidneys.
- Mycotoxins have a significant negative impact on the poultry and swine defense mechanisms and immune system.
- For example, fumonisins & deoxynivalenol (DON) predispose to the development of necrotic enteritis in broilers.

In today's environment, the presence of mycotoxins is an inherent risk. Chances are high that multiple mycotoxigenic molds such as *Aspergillus, Fusarium* and *Penicillium* and the related toxins creep into silos and corn silage at harvest and after ensiling. A recent survey highlighted that 99% of grain samples collected contained mycotoxins and over 85% contained multiple mycotoxins.

More than 400 types of mycotoxins have been identified so far, also diverse in their chemistry and effect on animals. Feed contaminated with mycotoxins causes a broad spectrum of problems ranging from reduction in feed intake and growth performance to compromised reproduction, health and immunity. Symptoms are often non-specific and cost the agricultural sector billions of dollars per annum.

Feed dietary mycotoxins can also end up in animal products destined for human consumption such as milk, eggs and meat where they remain as stable and inert toxic molecules but their fate is unknown once within the human body.

Under normal conditions multiple-mycotoxin contamination is likely. Multiple mycotoxins can have a synergistic or additive negative effect, increasing the overall negative impact on animal's performance and health. Mycotoxins from fungi in feed combined with bacterial toxins further increase the negative health issues.

Mycotoxins may also occur in a conjugated form either soluble (masked or modified mycotoxins produced by fungi and/or plants) or incorporated onto/associated with/attached to macromolecules (bound mycotoxins).

That effectively means that not only 'parent' mycotoxins but also their fungal and plant metabolites need to be quantified precisely for an appropriate feed risk assessment. However, individual raw material (grains/seeds) and/or feed sampling for mycotoxin analysis, although a critical factor, is often overlooked due to the high cost and the relative long (analysis) time involved. Moreover, the sampling itself of raw materials constitutes a major limitation due to the existence of what is known as 'hot' spots and the estimated relevant error occurrence during the sampling procedure is ~88%. Additionally, mycotoxin analysis of the raw materials does not necessarily warrant a true representation of the final feed that the animal consumes, as more mycotoxin transformations may take place during storage. Also, producers often rely on quick and often cheap(er) analysis tools for mycotoxin detection such as Lateral Flow Devices (LFDs) and ELISAs. While most of the LFDs provide qualitative results, some more recent versions of LFDs and all ELISAs provide quantitative results. However, both methods are limited in the fact that a) cross-reactivity may occur which in turn can invalidate the results and thereby, impact scientific reproducibility and that b) only one mycotoxin can be detected at a time and that not all tests are fit for different types of feed. Thus, this approach proves either insufficient or uneconomical for a full feed risk assessment.

More importantly, inherently, the feed risk assessment approach *per se* does not provide a true representation of the true exposure of animals to mycotoxins. Only methodological approaches that aim/allow a) the detection of several mycotoxins and their Phase I and Phase II metabolites b) with high analytical precision, c) in biological fluids in animals and d) with a practical, user-friendly as well as easy to sample and transport method can provide such meaningful information. It is only then when someone can get a true representation of the mycotoxins and their metabolites that systemically enter and expose the animal.

Reference is hereby made to a Research Paper entitled "Multi-mycotoxin analysis using dried blood spots and dried serum spots" by B. Osteresch, S. Viegas, B. Cramer and H-U Humpf in Anal. Bioanal. Chem (2017) 409:3369-3382; (Osteresch et al. 2017)

This study discloses a multi-mycotoxin approach for biomonitoring and quantification of 27 important mycotoxins and mycotoxin metabolites in human blood samples.

For a.o. Dried Blood Spots (DBS), HPLC-MS/MS detection method is used.

The study establishes a validated multi-mycotoxin approach for the detection of 27 mycotoxins and metabolites in dried blood/serum spots based on a fast sample preparation followed by sensitive HPLC-MS/MS analysis.

However, this study deals exclusively with the application of the methodology to human applications and transfer of the proposed methodology to animals cannot be taken for granted.

In a first paragraph, in the introduction pg. 3370, it is stated that Dried Blood Spots (DBS) are suitable for extensive biomonitoring studies of environmental contaminants in humans or animals. More specifically, reference is made to study nr. 14 by Batterman S, Chernyak S, entitled "Performance and storage integrity of dried blood spots for PCB, BFR and pesticide measurements", Sci Total Environ. 2014;494-495:252-60. This study investigates the suitability of DBS analyses for population studies of exposure to three chemical groups: polychlorinated biphenyls (PCBs), brominated flame retardants (BFRs), and chlorinated pesticides.

The collection of whole blood samples on paper, known as dried blood spot (DBS), dates back to the early 1960s in human newborn screening for inherited metabolic disorders. The last five years, DBS-LC-MS/MS has emerged as an important method for quantitative analysis of several molecules/analytes. The DBS-LC-MS/MS approach has been expanded for different types of analytes in the human sector such as polychlorinated biphenyls (PCBs), Brominated flame retardants (BFRs), pesticides (Batterman S and Chernyak S, 2014), hormones (Thomas and Thevis, 2018) and drugs (Wickremsinhe et al. 2018). However, the DBS-LC-MS/MS approach has not, yet, been adopted within the veterinary sector and no prior application for the detection of mycotoxins in farming animals is known to date. Several parameters need to be checked and validated for appropriate adoption on a particular animal species such as analytical sensitivity due to the small sample size, knowledge of the animal species-specific *in vivo* metabolism of mycotoxins to determine the most appropriate biomarker, potential impact of various blood sample properties on accurate quantification, sample quality, the impact of blood hematocrit, analyte stability, within day analytical variation, between days analytical variation etc.

In that context, it should be noted that this study by Batterman S, Chernyak S (2014) referenced in the introduction of this Research Paper refers to the detection of pesticides and other environmental toxic contaminants via DBS in animals, but not *per se* to mycotoxins. The transfer of the DBS concept for the detection of the environmental contaminants such as pesticides and heavy metals to mycotoxins, is neither simple nor evident or obvious.

In support hereof, it should be noted that PCBs, BFRs, and several classes of manmade chemicals such as pesticides are typically 'organo-synthetic' structures. Namely not naturally occurring structures as they carry one or more chlorine, bromine, fluorine (or other) atoms of non-naturally occurring elements within organic based structures.

As a result, not only their corresponding physicochemical properties but also their stability in DBS by application in paper based Find The Agent (FTA) cards may be totally different to that of mycotoxins, the latter being solely naturally occurring (organic) molecules. In fact, the authors Batterman & Chernyak, report that the DBS method proved stable at room temperature for pesticides and PCBs but not for Polybrominated Diphenyl ethers (PBDEs).

Differently phrased, one needs to perform substantive research efforts in order to determine:
- which mycotoxins and their specific (Phase I & Phase II) metabolites should be targeted and bio-monitored;
- for a specific animal species and
- for a specific biological sample (urine, blood, feces, excreta etc.).
as this relates directly to their unique metabolic pathway for a specific animal species.

Further, even if satisfactorily responses to the above questions are at hand, there still remains the need for a reliable, cost-effective means and method so as to detect in a qualitative and quantitative manner the presence of such mycotoxins and its metabolites in a sample of the selected animal to be bio-monitored.

Until today, mycotoxins are determined in the animal production farming field only in feed. Feed analysis provides only a rough estimate of the risk involved in relation to the amount of mycotoxins that animals could be exposed to. Moreover, feed analysis is prone to significant methodological errors due to the presence of hotspots and the difficulty of determining masked mycotoxins, both leading to underestimation of the risk. Therefore, although the feed risk assessment still remains a tool, its real usefulness, when applied in isolation, remains highly questionable as it lacks vital information with regards to the true exposure of animals to mycotoxins.

Additionally, routine mycotoxin biomonitoring methods do not include mycotoxin phase I and phase II metabolites. This may significantly underestimate mycotoxin exposure especially for heavily metabolized mycotoxins. Additional research efforts are also needed to measure metabolites *in vivo* after exposure and to establish which mycotoxin metabolites should be prioritized for the inclusion during biomonitoring efforts.

As a result, there remains a clear need for a cost effective and reliable method for determining the true exposure of mycotoxins to animals, broiler chicken and pigs in particular.

### References:

(1) Batterman S, Chernyak S Performance and storage integrity of dried blood spots for PCB, BFR and pesticide measurements, Sci Total Environ. 2014;494-495:252-60
(2) Thomas A, Thevis M, Analysis of insulin and insulin analogs from dried blood spots by means of liquid chromatography-high resolution mass spectrometry. Drug Testing and Analysis. 2018; doi.org/10.1002/dta.2518
(3) Lauwers, Marianne et al. 2019. "Multi LC-MS/MS and LC-HRMS Methods for Determination of 24 Mycotoxins Including Major Phase I and II Biomarker Metabolites in Biological Matrices from Pigs and Broiler Chickens." Toxins 11(3): 171.
(4) Osteresch, Bernd, Susana Viegas, Benedikt Cramer, and Hans Ulrich Humpf. 2017. "Multi-Mycotoxin Analysis Using Dried Blood Spots and Dried Serum Spots." Analytical and Bioanalytical Chemistry 409(13): 3369-82. http://www.ncbi.nlm.nih.gov/pubmed/28299415 (February 13, 2020).
(5) Wickremsinhe, Enaksha R. et al. 2018. "Incorporating Dried Blood Spot LC-MS/MS Analysis for Clinical Development of a Novel Oncolytic Agent." Bioanalysis 10(5): 341-56.

In the referenced article (3) above, a method for detecting up to 24 mycotoxins including major phase I and II biomarker metabolites has been described.

This method comprises a three-step process, including:
1) first subjecting a (first part of a) DBS sample to an LC-MS/MS procedure, the MS spectrometer operating in electrospray positive mode,
2) followed by subjecting a further (part of a) DBS sample to an LC-MS/MS procedure, the MS spectrometer operating in electrospray negative mode, and
3) finally subjecting a still further (part of a) DBS sample to an LC-HRMS procedure.

In the method described in this article, the use of the HRMS process and apparatus is required for detecting i.a. the phase II metabolites.

Although this method may be suitable for research purposes, the necessary use of an HRMS spectrometer renders this method not suitable for use in real-field applications for the following reasons:
1) the raw data files generated by a LC-HRMS instrument are very large, implying that as a result sample processing takes a lot of time, unpractical for use in daily screening
2) HRMS analysis requires the use of a dedicated HRMS spectrometer, which is a different apparatus as compared to a (non-HR or classical) MS spectrometer; so, in order to perform the method described in this article, apart from the liquid chromatograph tandem mass spectrometer, the operator needs to search for and submit the sample to a different apparatus, which, in view of its high cost and complicated processing, not readily available;
3) the overall process screening time required to process a DBS sample in this three-step process is quite long: the first run in ESI positive mode on the LC-MS/MS instrument takes around 16 minutes, the further run in ESI negative mode on the LC-MS/MS instrument takes a further 12 min, and the additional run on the LC-HRMS instrument, also in ESI negative mode, takes another 12 min; so, all in total a processing time of at least 40 min (16+12+12) is required;
4) finally, the method as described only allows for the detection of 24 analytes, which for multi-mycotoxin biomonitoring often is insufficient.

So there remains a need for an efficient multi-mycotoxin biomonitoring screening method, suitable for real-life conditions.

### Objectives of the invention

The aim of the inventors is to find novel ways to evaluate the systemic exposure of feed mycotoxins and their intermediates on animals.

Routine mycotoxin biomonitoring methods do not include mycotoxin phase I and phase II metabolites and this may significantly underestimate mycotoxin exposure especially for heavily metabolized mycotoxins.

The primary aim and objective of the invention is to provide a) adequate methods and b) cheap, easy and user-friendly means that focus on animal mycotoxin exposure assessment.

In that context, the main objectives of the invention are to identify novel biomarkers of exposure and to provide appropriate analytical tools for the precise determination and quantification not only of mycotoxins but also of their relevant metabolites *in vivo* after exposure and to establish which mycotoxin metabolites should be prioritized for the inclusion during biomonitoring efforts for chickens or pigs.

A further objective of the invention is that such biomonitoring method and means may provide more reliable data related to the animal's true impact via contaminated feed and in combination with a number of biotic and abiotic stress factors (described previously) *in situ* i.e. under real farming conditions.

It is thus believed that the invention has a great potential for use as a diagnostic tool with significant economic impact in industrial animal farming.

These objectives and advantages are given only by way of illustrative example, and such objectives may be exemplary of one or more embodiments of the invention. Other desirable objectives and advantages inherently achieved by the disclosed invention may occur or become apparent to those skilled in the art.

In view of the above, there remains a need for a reliable and cost-efficient biomonitoring method for determining the exposure of animals to multi-mycotoxins, in particular broiler chicken or pigs.

### Statement of invention:

The present inventors have conducted extensive studies in order to solve the above-mentioned problems.

These studies have resulted in the invention as described hereinafter.

The inventors have successfully found a method for biomonitoring mycotoxins and their relevant phase I and phase II metabolites in the blood of broiler chickens or pigs as described hereinafter.

The invention is set forth and characterized in the main claim, while the dependent claims describe other characteristics and specific features for preferred embodiments of the invention.

According to one aspect of the invention, there is provided a method for the detection of the following (10) mycotoxins and metabolites in broiler chickens or pigs,
- Aflatoxin B1 (AFB1),
- Aflatoxin B2 (AFB2),
- Aflatoxin M1 (AFM1),
- Fumonisin (FB1),
- Fumonisin (FB2),
- Ochratoxin A (OTA),
- Deoxynivalenol (DON),
- T2 toxin (T2),
- HT-2-toxin (HT2),
- Zearalenone (ZEN),
said method comprising:
- collecting the blood of broiler chickens or pigs as a dried blood sample;
- preparing the dried blood sample for analysis;
- analyzing the prepared dried blood sample by liquid chromatography-tandem mass spectrometry (LC-MS/MS) in two steps:
   a) detecting in a LC-MS/MS step,
      - the reversed phase LC process using as mobile phase, a mixture of acetic acid, acetonitrile and water, the proportion of the acetonitrile solution over the water solution gradually increasing during the process, and at the end diminishing to recondition the LC column,
      - the mass spectrometer operating in negative electro-spray ionization mode,
         and
   b) detecting in another LC-MS/MS step,
      - the reversed phase LC process using as mobile phase, a mixture of ammonium formate, formic acid, water and methanol, the proportion of the methanol solution over the water solution gradually increasing during the process, and at the end diminishing to recondition the LC column,
      - the mass spectrometer operating in positive electro-spray ionization mode.

So, the method of the present invention comprises a two-step process, whereby in each step a duly prepared (part of a) dried blood sample is subjected to a liquid chromatography tandem mass spectrometry (LC-MS/MS) procedure.

In a preferred method of the present invention, the following (8) additional mycotoxins and metabolites are detected simultaneously:
- Alternariol methyl ether (AME),
- Alternariol (AOH),
- Tenuazonic Acid (TEA),
- Beauvericin (BEA),
- Enniatin A, A1, B, B1 (ENNA, ENNA1, ENNB, ENNB1).

In a still further preferred method of the present invention, the following (18) additional mycotoxins and metabolites are detected simultaneously:
- de-epoxy-deoxynivalenol (DOM1),
- 15-acetyldeoxynivalenol (15ADON),
- 3-acetyldeoxynivalenol (3ADON),
- Zearalanone (ZAN),
- α-Zearalenol (AZEL),
- α-Zearalanol (AZAL),
- β-Zearalanol (BZAL),
- β-Zearalenol (BZEL),
- DON-glucuronide (DON-GlcA),
- DON-Sulphate (DON-S),
- ZEN-glucuronide (ZEN-GlcA),
- ZEN-sulfate (ZEN-S),
- α-zearalenol-glucuronide (A-ZEL-GlcA),
- β-zearalenol-glucuronide (B-ZEL-GlcA),
- Fumonisin B3 (FB3),
- Aflatoxin M2 (AFM2),
- Aflatoxin G 1 (AFG1),
- Aflatoxin G2 (AFG2).

According to the latter preferred embodiment of the present invention, all in total 36 mycotoxins, including some of its phase I and phase II metabolites, are detected simultaneously in the two run-method of the present invention.

The term 'detection' in the context of the present invention should be understood as on the one hand the (qualitative) determination of the presence of certain compounds and - at least as the mycotoxins and some of their phase I metabolites are concerned - on the other hand the (quantitative) calculation of the concentration of these compounds. According to a preferred embodiment of the invention, the compounds subject of the detection according to the method of the present invention are some specified mycotoxins and some of their phase I and phase II metabolites, as defined hereinafter.

### Detailed description of the invention.

### Collection and preparation of the dried blood sample (DBS)

According to a preferred embodiment of the invention, collecting the dried blood sample comprises collecting a drop of blood on a filter paper, followed by drying at room temperature.

According to a further preferred embodiment of the invention, collecting the dried blood sample comprises isolating the dried blood sample from the filter paper by punching out a paper disk out of the filter paper, preferably round and about 8 mm in diameter, using a biopsy punch.

According to a further preferred embodiment of the invention, prior to analyzing, the blood from the dried blood sample is prepared for analysis, such preparation including extracting the dried blood sample in an extraction solvent.

The extraction solvent preferably comprises a water/acetonitrile/acetone mixture. Thereupon, the extraction solvent is dried and reconstituted in a reconstitution solvent, such reconstituting solvent preferably comprising a water/methanol/formic acid mixture.

So, the collected dried blood sample is extracted in an extraction solvent, dried, reconstituted in a reconstitution solvent, whereupon the reconstituted dried blood sample can be analyzed by liquid chromatography tandem mass spectrometry.

### LC-MS/MS

The basic analytical technique used in the method of the present invention is liquid chromatography with tandem mass spectrometry, hereinafter abbreviated as LC-MS/MS.

The term Liquid Chromatography is hereinafter referred to as LC.

The term Mass Spectrometry is hereinafter referred to as MS.

The term tandem Mass Spectrometry is hereinafter referred to as MS/MS.

The term High Resolution Mass Spectrometry is hereinafter referred to as HR-MS.

The term **"Liquid Chromatography (LC)"** as used in the context of the present invention should be understood as a laboratory technique for the separation of a mixture of compounds. The mixture is dissolved in a fluid called the *mobile phase,* which carries it through a structure holding another material called the *stationary phase.* The various constituents of the mixture travel at different speeds, causing them to separate. The separation is based on differential partitioning between the mobile and stationary phases. Subtle differences in a compound's partition coefficient result in differential retention on the stationary phase and thus affect the separation.

Chromatography may be preparative or analytical. The purpose of preparative chromatography is to separate the components of a mixture for later use, and is thus a form of purification. Analytical chromatography is done with smaller amounts of material and is for establishing the presence or measuring the relative proportions of analytes in a mixture. For the purpose of the present invention, analytical chromatography is applied.

Tables 4 and 5, included further in the present specification, illustrate the above: the retention times are set forth for the various components comprised in the DBS sample subjected to the LC process step as comprised in the method of the present invention.

The term **"Mass Spectrometry (MS)"** as used in the context of the present invention should be understood as an analytical technique that measures the mass-to-charge ratio of ions. The results are typically presented as a mass spectrum, a plot of intensity as a function of the mass-to-charge ratio. In the present case, mass spectrometry is applied to a quite complex organic mixture.

A mass spectrum is a plot of the ion signal as a function of the mass-to-charge ratio. The spectra are used to determine the elemental or isotopic signature of a sample, the masses of particles and of molecules, and to elucidate the chemical identity or structure of molecules and other chemical compounds.

In the MS procedure as applied in the present invention, a sample, being the collected dried blood sample, extracted, dried and reconstituted as described below, is ionized. This will cause some of the sample's molecules to break into charged fragments or simply become charged without fragmenting. These ions are then separated according to their mass-to-charge ratio, for example by accelerating them and subjecting them to an electric or magnetic field: ions of the same mass-to-charge ratio will undergo the same amount of deflection. The ions are detected by a mechanism capable of detecting charged particles, such as an electron multiplier. Results are displayed as spectra of the signal intensity of detected ions as a function of the mass-to-charge ratio. The molecules in the dried blood sample are then identified either through a characteristic pattern for the whole of the mycotoxin or phase I metabolite molecules or through a characteristic pattern for the fragments of the mycotoxin or metabolite molecules.

### LC-MS/MS

The term "Liquid Chromatography tandem Mass Spectrometry (LC MS/MS)" as used in the context of the present invention should be understood as Liquid Chromatography, followed by Mass Spectrometry. In such a case the Dried Blood Sample, after being extracted in the extraction solvent, being dried and reconstituted in the reconstituting solvent, is injected in the column of the Liquid Chromatography apparatus; the various mycotoxins and phase I metabolites, exiting the Liquid Chromatography column, are then subjected to an identification and quantification analysis by a Mass Spectrometry apparatus, based on a targeted approach.

Liquid Chromatography with tandem mass spectrometry (LC-MS/MS) is a powerful analytical technique that combines the separating power of liquid chromatography with the highly sensitive and selective mass analysis capability of triple quadrupole mass spectrometry.

A sample solution containing analytes of interest are pumped through a stationary phase (LC column) by a mobile phase flowing through at high pressure. Chemical interaction between the components of the sample, the stationary phase and the mobile phase affects different migration rates through the LC column affecting a separation. The wide variety of stationary phase and mobile phase combinations allow for customizing a separation to suit many complex solutions.

After elution from the LC column, the effluent is directed to the mass spectrometer. The mass spectrometer for an LC-MS/MS system has an ionization source where the LC column effluent is nebulized, desolvated and ionized creating charged particles. These charged particles then migrate under high vacuum through a series of mass analyzers (quadrupole) by applying electromagnetic fields. A specific mass/charge precursor ion (or parent ion) is targeted to pass through the first quadrupole, excluding all other mass/charge ratio particles. In the collision cell, the selected mass/charge ions are then fragmented into product ions (or daughter ions) by collision with an inert gas. The third quadrupole is used to target specific product ion fragments. The resulting isolated product ions are then quantified with an electron multiplier. This transition of ions from the precursor to product ion (also referred to as MS²) is highly specific to the structure of the compound of interest and therefore provides a high degree of selectivity.

The strength of this technique lies in the separation power of LC for a wide range of compounds combined with the capability of the MS to quantify compounds with a high degree of sensitivity and selectivity based on the unique mass/charge (m/z) transitions of each compound of interest.

### 36 parent mycotoxins and phase I & II metabolites

As set forth supra, a preferred embodiment of the present invention is directed to the detection of one or more of the 36 mycotoxins and their major phase I & II metabolites, set forth in the following list. In this list, the various compounds are grouped into either mycotoxins (parent), phase I metabolites, and phase II metabolites.

Also, the corresponding compounds detectable by the method of the prior art, described in the abovementioned reference (3) are also included in this table. Finally the table indicates whether under the method of the present invention, the indicated compound is detected by the mass spectrometer either operating in positive (+) or negative (-) electrospray ionization (ESI-) mode.

Finally, the same table indicates whether according to the method of the present invention, as compared to the prior art method described in reference article (3), the indicated compound is detected quantitatively or qualitatively.

**Table I List of Mycotoxins detected in DBS by LC-MS/MS**

| | **Prior art list 23 Analytes** | **List acc. to invention 36 Analytes** | **ESI-Mode** |
|---|---|---|---|
| **Mycotoxins (parent) fully quantified** | | **Mycotoxins (parent) fully quantified** | |
| **1** | Deoxynivalenol (DON) | Deoxynivalenol (DON) | + |
| **2** | 3/15-acetyl deoxynivalenol (3/15ADON) | 3-acetyl deoxynivalenol (3/ADON) | + |
| **3** | Aflatoxin B1 (AFB1) | Aflatoxin B1 (AFB1) | + |
| **4** | Enniatin A (ENNA) | Enniatin A (ENNA) | + |
| **5** | Enniatin A1 (ENNA1) | Enniatin A1 (ENNA1) | + |
| **6** | Enniatin B (ENNB) | Enniatin B (ENNB) | + |
| **7** | Enniatin B1 (ENNB1) | Enniatin B1 (ENNB1) | + |
| **8** | Beauvericin (BEA), | Beauvericin (BEA), | + |
| **9** | Fumonisin B1 (FB1) | Fumonisin B1 (FB1) | + |
| **10** | Fumonisin B2 (FB2) | Fumonisin B2 (FB2) | + |
| **11** | Ochratoxin A (OTA) | Ochratoxin A (OTA) | + |
| **12** | T-2 toxin (T-2) | T-2 toxin (T-2) | + |
| **13** | Zearalenone (ZEN) | Zearalenone (ZEN) | - |
| **14** | Tenuazonic acid (TEA) | Tenuazonic acid (TEA) | - |
| **15** | Alternariol (AOH) | Alternariol (AOH) | - |
| **16** | Alternariol mono-methylether (AME) | Alternariol mono-methylether (AME) | - |
| **17** | | Aflatoxin B2 (AFB2) | - |
| **18** | | Aflatoxin G1 (AFG1) | - |
| **19** | | Aflatoxin G2 (AFG2) | - |

| | **Phase I metabolites fully quantified** | **Phase I metabolites fully quantified** | |
|---|---|---|---|
| **1** | de-epoxy-Deoxynivalenol (DOM1) - phase I metabolite | de-epoxy-Deoxynivalenol (DOM1) - phase I metabolite | + |
| **2** | Aflatoxin M1 (AFM1) - phase I metabolite | Aflatoxin M1 (AFM1) - phase I metabolite | + |
| **3** | α-Zearalenol (AZEL) - phase I metabolite | α-Zearalenol (AZEL) - phase I metabolite | - |
| **4** | β-Zearalenol (BZEL) - phase I metabolite | β-Zearalenol (BZEL) - phase I metabolite | - |
| **5** | α-Zearalanol (AZAL) - phase I metabolite | α-Zearalanol (AZAL) - phase I metabolite | - |
| **6** | β-Zearalanol (BZAL) - phase I metabolite | β-Zearalanol (BZAL) - phase I metabolite | - |
| **7** | Zearalanone (ZAN) - phase I metabolite | Zearalanone (ZAN) - phase I metabolite | - |

| | | | |
|---|---|---|---|
| **Phase II metabolites only qualified: present/absent** | | **Phase II metabolites only qualified: present/absent** | |
| **1** | | 15-acetyl deoxynivalenol (15ADON) | + |
| **2** | | DON-glucuronide (DON-GlcA) | + |
| **3** | | DON-sulphate (DON-S) | + |
| **4** | | ZEN-glucuronide (ZEN-GlcA) | + |
| **5** | | a-ZEL-glucuronide (AZEL-GlcA) | - |
| **6** | | b-ZEL-glucuronide (BZEL-GlcA) | - |
| **7** | | ZEN-sulfate (ZEN-S) | + |
| **8** | | Fumonisin B3 (FB3) | - |
| **9** | | Aflatoxin M2 (AFM2) | - |
| **10** | | HT-2 toxin (HT-2) | + |

Near to the name of each mycotoxin or phase I or II metabolite, the above table comprises also the name in abbreviated form (such as e.g. DON for Deoxynivalenol). In the description that follows, reference to the above components may be made by referring to such abbreviated name.

So, as compared to the LC-MS/MS part of the prior art technique disclosed in the article referenced above as (3), three additional parent mycotoxins can be detected by the method of the present invention.

Further, by applying the method of the present invention, 10 phase II metabolites can be detected in the LC-MS/MS method, without any need to apply the complicated LC-HRMS technique, as was the case for the prior art technique disclosed in reference article (3).

### ESI positive and negative mode

In the method of the present invention the overall procedure for analyzing a DBS sample is as follows:
a) a first part of a given sample is submitted to a first LC step, the mobile phase in the LC apparatus being the mixture as set forth in Table 2 below (phase A - water + phase B methanol) followed by an MS procedure, operating in ESI positive mode;
   this yields results for the analytes listed in Table 4; (21 analytes);
b) hereupon the remaining part of the same sample (or alternatively a separate identical sample) is submitted to a second LC step, the mobile phase in the LC apparatus being the mixture as set forth in Table 3 (phase A - water + phase B acetonitrile) again followed by an MS procedure, operating in ESI Negative mode;
this yields results for the analytes listed in Table 5; (15 analytes).

The sequence as set forth above, whereby in the first step the MS operates in ESI positive mode, and whereby in the second step the MS operates in ESI negative mode, can be reversed. These is no technical requirement to have the MS apparatus work either in positive or negative mode in the first step; any of both these steps (ESI +, resp. ESI -) can be applied first, whereupon in the second step the other mode is used (ESI -, resp. ESI +).

So, what is applied in the method of the present invention is LC/MS step 1, followed by LC/MS step 2. Altogether, this 2-step-procedure takes 23 minutes for a complete analysis and detection.

**Table 2. UPLC conditions for analytes determined in dried blood spots (DBS) using the ESI positive mode.**

| | | | |
|---|---|---|---|
| **Column + guard column** | Acquity HSS-T3; column: 100 × 2.1 mm i.d., dp: 1.8 µm; | | |
| | VanGuard pre-column: 5 × 2.1 mm i.d., dp: 1.8 µm | | |
| **Column temperature** | 40 °C | | |
| **Sample manager temperature** | 8 °C | | |
| **Gradient program** | | | |
| ***Mobile phase A*** | 0.3 % formic acid + 10 mM ammonium formate in water | | |
| ***Mobile phase B*** | 0.3 % formic acid + 10 mM ammonium formate in methanol | | |

| ***Time (min)*** | ***Flow rate (ml*/*min)*** | ***% mobile phase A*** | ***% mobile phase B*** |
|---|---|---|---|
| 0.0 | 0.3 | 95 | 5 |
| 0.5 | 0.3 | 95 | 5 |
| 1.5 | 0.3 | 40 | 60 |
| 2.5 | 0.3 | 40 | 60 |
| 5.0 | 0.3 | 20 | 80 |
| 6.0 | 0.3 | 1 | 99 |
| 8.9 | 0.3 | 1 | 99 |
| 9.0 | 0.3 | 95 | 5 |
| 12.0 | 0.3 | 95 | 5 |

As shown by the above table, the proportion of the methanol solution over the water solution gradually increases during the LC process. At the end, this trend is reversed, the proportion of the water solution over the methanol solution being increased again so as to recondition the LC column.

As compared to the prior art process disclosed in reference (3), the inventors have succeeded in reducing the overall process time to only 12 minutes (as compared to a required process time of 16 minutes in the prior art.)

**Table 3. UPLC conditions for analytes determined in DBS using the ESI negative mode.**

| | | | |
|---|---|---|---|
| **Column + guard column** | Acquity HSS-T3; column: 100 × 2.1 mm i.d., dp: 1.8 µm; | | |
| | Vanguard pre-column: 5 × 2.1 mm i.d., dp: 1.8 µm | | |
| **Column temperature** | 40 °C | | |
| **Sample manager temperature** | 8 °C | | |
| **Gradient program** | | | |
| ***Mobile phase A*** | 1 % acetic acid in water | | |
| ***Mobile phase B*** | **1 % acetic acid in acetonitrile** | | |

| ***Time (min)*** | ***Flow rate (ml*/*min)*** | ***% mobile phase A*** | ***% mobile phase B*** |
|---|---|---|---|
| 0.0 | 0.3 | 95 | 5 |
| 1.0 | 0.3 | 95 | 5 |
| 4.5 | 0.3 | 40 | 60 |
| 7.8 | 0.3 | 40 | 60 |
| 8.0 | 0.3 | 95 | 5 |
| 11.0 | 0.3 | 95 | 5 |

As shown by the above table, the proportion of the acetonitrile solution over the water solution gradually increases during the LC process. At the end, this trend is reversed, the proportion of the water solution over the acetonitrile solution being increased again so as to recondition the LC column.

As compared to the prior art process disclosed in reference (3), the inventors have succeeded in reducing the overall process time to only 11 minutes (as compared to a required process time of 12 minutes in the prior art.)

The term 'parent' mycotoxin as used in the above table and throughout the present specification means a mycotoxin that can be found in the (animal) feed, this to make the difference with a metabolite of such mycotoxin that can be formed in the animal (for example in the liver).

### MS/MS ESI + and ESI - mode

Upon preparation of the DBS sample, the actual LS-MS/MS two-step process according to the present invention, can be applied.

The two subsequent steps are:
- in one step, analyzing by LC-MS/MS, the mass spectrometer apparatus operating in negative electro-spray ionization (ESI-) mode, and using as mobile phase, a mixture of acetic acid, water and acetonitrile, the proportion of acetonitrile gradually increasing during the liquid chromatography process, a first series of mycotoxins and its phase I & II metabolites:
   - in another step, analyzing by LC-MS/MS, the mass spectrometer apparatus operating in positive electro-spray ionization mode, and using as mobile phase, a mixture of ammonium formate, formic acid, water and methanol, the proportion of methanol gradually increasing during the liquid chromatography process, a further series of mycotoxins and its phase I & II metabolites.

So, contrary to the prior art comprising detection of some phase II metabolites of mycotoxins by LC-HRMS, the method of the present invention comprises detection of such phase II metabolites of mycotoxins by the usual LC-MS/MS method.

**Table 4.**

| Analytes determined in DBS using the ESI positive mode: | | | |
|---|---|---|---|
| LC retention time, Internal standard, Quantitative (Quan) or Qualitative (Qual) detection | | | |
| Name | Retention time (min) | Internal standard | Quan/Qual |
| DON | 3.27 | [¹³C₁₅]-DON | Quan |
| DOM-1 | 3.54 | [¹³C₁₅]-DON | Quan |
| 15-ADON | 3.83 | [¹³C₁₅]-DON | Qual |
| 3-ADON | 3.82 | [¹³C₁₅]-DON | Quan |
| [¹³C₁₅]-DON | 3.27 | / | / |
| T-2 | 5.92 | [¹³C₂₄]-T2 | Quan |
| HT-2 | 5.29 | [¹³C₂₄]-T2 | Qual |
| [¹³C₂₄]-T2 | 5.92 | / | / |
| AFB1 | 4.47 | [¹³C₁₇]-AFB1 | Quan |
| AFB2 | 4.30 | [¹³C₁₇]-AFB1 | Quan |
| AFG1 | 4.05 | [¹³C₁₇]-AFB1 | Quan |
| AFG2 | 3.91 | [¹³C₁₇]-AFB1 | Quan |
| [¹³C₁₇]-AFB1 | 4.46 | / | / |
| AFM1 | 3.91 | [¹³C₁₇]-AFM1 | Quan |
| AFM2 | 3.78 | [¹³C₁₇]-AFM1 | Qual |
| [¹³C₁₇]-AFM1 | 3.91 | / | / |
| OTA | 6.42 | [¹³C₂₀]-OTA | Quan |
| [¹³C₂₀]-OTA | 6.41 | / | / |
| ENNA | 8.50 | [¹⁵N₃]-ENNB or [¹³C₂₀]-OTA | Quan |
| ENNA1 | 8.38 | [¹⁵N₃]-ENNB or [¹³C₂₀]-OTA | Quan |
| ENNB | 8.13 | [¹⁵N₃]-ENNB or [¹³C₂₀]-OTA | Quan |
| ENNB1 | 8.25 | [¹⁵N₃]-ENNB or [¹³C₂₀]-OTA | Quan |
| BEA | 8.21 | [¹⁵N₃]-ENNB or [¹³C₂₀]-OTA | Quan |
| [¹⁵N₃]-ENNB | 8.13 | / | / |
| FB1 | 5.38 | [¹³C₃₄]-FB1 | Quan |
| FB2 | 6.52 | [¹³C₃₄]-FB1 | Quan |
| FB3 | 6.05 | [¹³C₃₄]-FB1 | Qual |
| [¹³C₃₄]-FB1 | 5.39 | / | / |

**Table 5.**

| Analytes determined in DBS using the ESI negative mode: | | | |
|---|---|---|---|
| LC retention time, Internal standard, Quantitative (Quan) or Qualitative (Qual) detection | | | |
| Name | Retention time (min) | Internal standard | Quan/Qual |
| TEA | 5.82 | [¹³C₆¹⁵N]-TEA | Quan |
| AOH | 5.93 | [¹³C₆¹⁵N]-TEA | Quan |
| [¹³C₆¹⁵N]-TEA | 5.81 | / | / |
| AME | 7.10 | [¹³C₁₈]-ZEN | Quan |
| ZEN | 7.17 | [¹³C₁₈]-ZEN | Quan |
| A-ZEL | 6.48 | [¹³C₁₈]-ZEN | Quan |
| B-ZEL | 6.16 | [¹³C₁₈]-ZEN | Quan |
| ZAN | 7.09 | [¹³C₁₈]-ZEN | Quan |
| A-ZAL | 6.59 | [¹³C₁₈]-ZEN | Quan |
| B-ZAL | 6.09 | [¹³C₁₈]-ZEN | Quan |
| [¹³C₁₈]-ZEN | 7.17 | / | / |
| DON-S | 3.80 | [¹³C₁₈]-ZEN | Qual |
| DON-GlcA | 3.77 | [¹³C₁₈]-ZEN | Qual |
| ZEN-S | ? | [¹³C₁₈]-ZEN | Qual |
| ZEN-GlcA | 5.54 | [¹³C₁₈]-ZEN | Qual |
| A-ZEL-GlcA | 5.24 | [¹³C₁₈]-ZEN | Qual |
| B-ZEL-GlcA | 4.64 | [¹³C₁₈]-ZEN | Qual |

### Spiking

According to a preferred embodiment of the present invention, the method comprises spiking the collected dried blood sample with one or more internal standards.

According to a further preferred embodiment of the present invention, the internal standard(s) are, resp. are selected from the following list of 8 compounds:
- ¹³C15-deoxynivalenol,
- ¹³C17-aflatoxin B1,
- ¹³C20-ochratoxin A,
- ¹³C24-T2-toxin,
- ¹³C34-fumonisin B1,
- ¹⁵N3-enniatin B,
- ¹³C6¹⁵N-tenuazonic acid,
- ¹³C18-zearalenone.

### Practical use of the method according to the invention.

The method as set forth above, as well as any of the preferred embodiments of such method, can be used for screening and assessing the exposure of pigs and broiler chickens to feed contaminated with mycotoxins.

Further such method is also suitable for assessing the addition of mycotoxin detoxifying agents to animal feed.

Further aspects and advantages of the invention as well as of its preferred embodiments will appear from the following detailed description.

### Further detailed description of the invention

The term **"monitoring"** as used in the present specification is understood to mean determining the exposure of animals to mycotoxins with the use of so-called biomarkers of exposure.

The term **"biomarkers"** as used in the present specification is understood to mean molecules that are a measure for the exposure by the animal to the mycotoxins and their phase I and phase II metabolites, and that can be found in the biological matrices of the animals.

Two types of biomarkers should be distinguished:
- direct (exposure) and
- indirect (mechanism/effect) biomarkers.

The latter are mainly non-specific and associated with either the effect or the mechanism of mycotoxins. The effect-based biomarkers are even less specific than the mechanism-based. A typical example is the alteration in feed intake after administration of deoxynivalenol. The direct biomarkers are specific and directly linked to the exposure. This type of biomarker is often the mycotoxin itself or their phase I and II metabolites. In biomonitoring, the direct biomarkers are the preferred biomarkers.

The detection of biomarkers can occur in easily accessible matrices of the animal to be bio-monitored, such as blood, urine or feces.

In the context of the present invention, detection of the direct biomarkers or biomarkers for exposure, for example for rapidly absorbed mycotoxins, are to be detected in the blood.

Peak concentrations can be detected in the blood and the concentration in blood is directly related with the exposure. Plasma is thus a good matrix to measure mycotoxins a few hours after exposure.

The time at which mycotoxins are absorbed by the animal varies substantially between the various mycotoxins and between animal species. The inventors have determined after experiments that the optimal time slot for the detection of such mycotoxins, is to collect the dried blood sample after 30 minutes up to two hours after feeding, preferably approximately 1 hour after feeding the animal.

The experimental data for this preferential time frame are described in the following article by the inventors:
The Article entitled "Biomarkers for Exposure as a tool for efficacy testing of a mycotoxin detoxifier in broiler chickens and pigs", by Marianne Lauwers, Siska Croubels, Ben Letor, Christos Gougoulias and Mathias Devreese, published on March 28, 2019 by Toxins 2019, 11, 187; doi:10.3390/toxins 11040187, www.mdpi.com/journal/toxins.

### Mycotoxins and phase I & II metabolites:

It is known to the person skilled in the art that animal feed may comprise a quite high number of different kinds of mycotoxins and the related metabolites.

As a first step, comprised in the method of the present invention, a selection should be made as to which mycotoxins and related metabolites should be the subject of the monitoring or assessment operation.

The mycotoxins targeted in the multi-method of the present invention comprise the regulated groups, i.e. aflatoxins, ochratoxin A and several *Fusarium* mycotoxins and two groups of unregulated or emerging mycotoxins, i.e. *Alternaria* mycotoxins and selected *Fusarium* mycotoxins (enniatins and beauvericin).

The selection of the abovementioned mycotoxins and their phase I & II metabolites has been based on a careful study of the regulations as presently in force in the European Union, and the occurrence data in animal feed.

EFSA (European Food Safety Agency)-regulated mycotoxins in feed are AFB1, OTA, FB1+FB2, T2+HT2, ZEN and DON.

Non-regulated mycotoxins are e.g. ENNs, BEA, AOH, AME and TEA.

As a result, in the method according to the present invention at least the following (10) mycotoxins and its metabolites are detected:
- Aflatoxin B1 (AFB1),
- Aflatoxin B2 (AFB2),
- Aflatoxin M1 (AFM1),
- Fumonisin (FB1),
- Fumonisin (FB2),
- Ochratoxin A (OTA),
- Deoxynivalenol (DON),
- T2 toxin (T2),
- HT-2-toxin (HT2),
- Zearalenone (ZEN).

According to a preferred embodiment, the method of the present invention comprises the detection of the following (8) additional mycotoxins and its metabolites:
Alternariol methyl ether (AME),
- Alternariol (AOH),
- Tenuazonic Acid (TEA),
- Beauvericin (BEA),
- Enniatin A, A1, B, B1 (ENNA, ENNA1, ENNB, ENNB1).

According to a further preferred embodiment, the method of the present invention comprises the detection of the following (18) additional mycotoxins and its metabolites:
- de-epoxy-deoxynivalenol (DOM1),
- 15-acetyldeoxynivalenol (15ADON),
- 3-acetyldeoxynivalenol (3ADON),
- Zearalanone (ZAN),
- α-Zearalenol (AZEL),
- α-Zearalanol (AZAL),
- β-Zearalanol (BZAL),
- β-Zearalenol (BZEL),
- DON-glucuronide (DON-GlcA),
- DON-Sulphate (DON-S),
- ZEN-glucuronide (ZEN-GlcA),
- ZEN-sulfate (ZEN-S),
- α-zearalenol-glucuronide (A-ZEL-GlcA),
- β-zearalenol-glucuronide (B-ZEL-GlcA),
- Fumonisin B3 (FB3),
- Aflatoxin M2 (AFM2),
- Aflatoxin G 1 (AFG1),
- Aflatoxin G2 (AFG2).

We will now describe in further detail the various steps of the method according to the invention:

### Production of the Dried Blood Sample.

The present invention is based on the use of Dried Blood Samples, given its convenience in production and ease of conservation and transport.

The Dried Blood Sample is produced by taking a drop of blood sample, transferring same to a saver card (paper card), preferably followed by punching out the central 8mm disk of the card.

The drop of blood can be taken from any part of the pig or broiler chicken, but preferably the blood is taken from the ear of the pig or from the leg of the broiler chicken.

As a next step, the drop of blood is transferred to a saver card.

As saver card, use can be made e.g. of a protein saver card marketed under the brand name "Whatman 903"^{®}, available from Sigma-Aldrich.

A sample collection area of the 903 Protein Saver Card contains five half-inch circles. Each circle holds 50 to 80 µl of sample.

The optimal volume to cover a complete circle on the saver card was determined as 60 µl. On the saver card, the drop of blood spreads out over the card and fills a circle of at least 8 mm. As a next step, the 8 mm central disk on the saver card is punched out with a biopsy punch.

The so punched out saver paper comprising (part of) the drop of blood, constitutes the dried blood sample hereinafter referred to as the DBS.

Based on the following experiments, it appears that irrespective of the specific amount of the drop of blood taken yields the same results in terms of qualification and quantification of the mycotoxins are obtained.

### Insertion of Internal Standards (IS) in the DBS

At this stage in the method according to the present invention, substances suitable to act as internal standard products are spiked into the DBS.

More in particular, according to a preferred mode of operation of the present invention, a combination of the following substances can be used as such internal standards and to that end are spiked into the DBS:

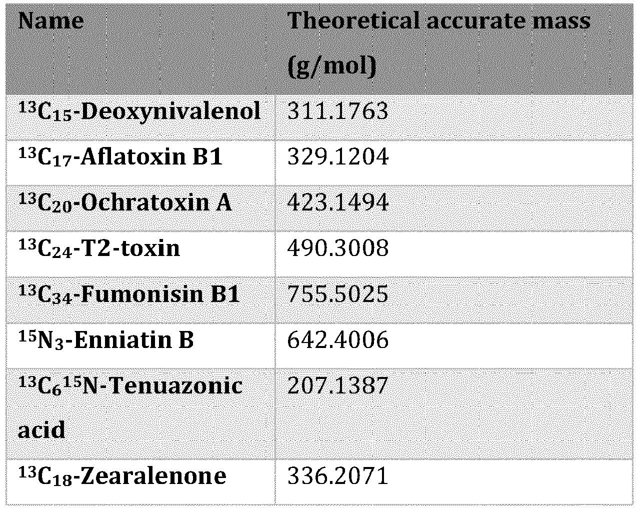

The abovementioned substances are suited to act as internal standards in the method of the present invention as they comprise either carbon atoms comprising 13 neutrons or nitrogen atoms comprising 15 neutrons and are as such easily detectable by the mass spectrometer and have very similar characteristics as the ¹²C and ¹⁴N components.

These substances are used as reference standards in the LC-MS/MS apparatus and in the HRMS apparatus used in the present invention.

Since an isotopically labeled Internal Standard (hereinafter referred to as IS) for each single mycotoxin is too expensive and not commercially available, an IS labeled with [¹³C] or [¹⁵N] was used for each group of mycotoxins, as follows:
[¹³C₁₅]-deoxynivalenol was used as IS for DON, DOM1 and 3/15ADON;
[¹³C₂₄]-T2-toxin for T2 and HT2;
[¹³C₁₇]-Aflatoxin B1 for AFB1, AFB2, AFG1, AFG2, AFM1 and AFM2;
[¹³C₂₀]-Ochratoxin A for OTA;
[¹³C₃₄]-Fumonisin B1 for FB1, FB2 and FB3;
[¹³C₆¹⁵N]-Tenuazonic acid for TEA, AME and AOH;
[¹³C₁₈]-Zearalenone for ZEN, AZAL, BZAL, AZEL, BZEL, ZAN, DON-GlcA, DON-S, ZEN-GlcA, ZEN-S, A-ZEL-GlcA and B-ZEL-GlcA;
[¹⁵N₃]-Enniatin B for ENNA, ENNA1, ENNB, ENNB1 and BEA.

Hence, an optimal correction for matrix effects and losses during sample preparation was obtained, which was confirmed during method validation.

### Extraction of the mycotoxins in a solvent

The next step in the method according to the invention is to extract the mycotoxins contained in the DBS, in a manner as pure and complete as technically feasible. Differently phrased, in this step of the method according to the invention, the aim is to isolate the mycotoxins in the DBS from as much of the other blood constituents as possible.

To this end, the method comprises placing the DBS in a test tube, e.g. made of glass, and adding a suitable extraction solvent.

According to a preferred mode of operation, as extraction solvent, use can be made of a mixture of water/acetone/acetonitrile, preferably in the following respective volumes: 30/35/35 v/v/v.

The amount of solvent to be used can vary widely, but preferably an amount ranging from 0.1 up to 10 ml, preferably from 0.5 up to 5 ml, more preferably around 1 ml can be used.

The sample comprising the DBS and the extraction solvent is subjected to an ultrasonic treatment, preferably during a period of time ranging from 15 to 45 minutes, preferable around 30 minutes.

Upon completion of the ultrasonic bath step, the solvent is poured over in another test tube; the remaining saver card paper can be removed.

### Drying and reconstitution of the solvent

The aim of the present step is to prepare a sample for injection into the liquid chromatography (LC) apparatus. This implies that the DBS sample should be further treated in view of the detectability as well in qualitative as in quantitative manner mycotoxins in the sample in the LC-MS/MS or LC-HRMS.

The latter can be realized on the one hand by an appropriate selection of the reconstitution solvent, and on the other hand by determining the appropriate amount of such reconstitution solvent to be used.

In this stage of the method of the present invention, the extraction solvent, containing the mycotoxins and related metabolites to be detected, is dried, e.g. under a gentle nitrogen stream at a suitable temperature, e.g. between 20 and 60 °C, preferably between 30 and 50 °C, more preferably at around 40 °C +/- 5 °C. Upon drying, the dried material is reconstituted in a solvent, suitable for injection into the LC apparatus.

The inventors have found that to that end, according to the method of the invention, a solvent consisting of water/methanol/formic acid preferably can be used. Preferably the dried material is reconstituted in an appropriate amount of such solvent, being 60 µl (this amount corresponding to the same amount of the initial DBS, drop of blood).

By selecting the same amount of blood as original DBS, the concentration of mycotoxins in the sample to be analyzed is the same as in the original blood drop.

### Experimental support:

The following experiments, performed by the inventors, illustrate the above.

Three different reconstitution solvents (n = 3 per condition) were evaluated: water/ACN/acetic acid (AA) (95/5/0.1, v/v/v); water/methanol /formic acid (60/40/0.1, v/v/v) and water/methanol (15/85, v/v). The first two mixtures have previously been applied for the detection of multiple mycotoxins or OTA alone in human DBS. The latter has been used by co-inventors Lauwers et al. in an LC-MS/MS method for multi-mycotoxin determination in pig and chicken plasma. The best results were obtained using methanol instead of acetonitrile in the reconstitution solvents (Figure 1). Next, calibration curves were made with the two methanol containing reconstitution solvents and the lowest concentration achieved for each component as well as the peak shape were compared. The mixture of water/methanol/formic acid (60/40/0.1, v/v/v) showed the best results for both parameters and was therefore chosen as the preferred solvent. The same findings were also observed for DBS obtained from broiler chickens.

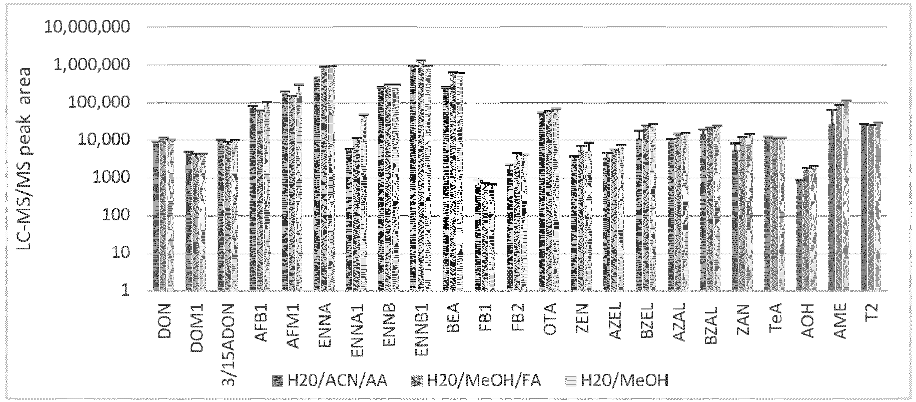

The above figure shows the evaluation of the analyte peak areas after LC-MS/MS analysis of DBS (60 µl), extracts which were re-dissolved in three different reconstitution solvents: water/acetonitrile (ACN)/acetic acid (AA) (95/5/0.1, v/v/v); water/methanol (MeOH)/formic acid (FA) (60/40/0.1, v/v/v) and water/MeOH (15/85, v/v). Individual mycotoxins were spiked in whole blood at a concentration of 10 ng·ml⁻¹. The mean (n = 3) LC-MS/MS peak areas + standard deviation (SD) is shown in graph.

The following mycotoxins were the subject of the experiments:
Deoxynivalenol (DON), de-epoxy-deoxynivalenol (DOM1), 3/15-acetyl deoxynivalenol (3/15ADON), aflatoxin B1 (AFB1), aflatoxin M1 (AFM1), enniatin A (ENNA), enniatin A1 (ENNA1), enniatin B (ENNB), enniatin B1 (ENNB1), beauvericin (BEA), fumonisin B1 (FB1), fumonisin B2 (FB2), ochratoxin A (OTA), zearalenone (ZEN), α-zearalenol (AZEL), β-zearalenol (BZEL), α-zearalanol (AZAL), β-zearalanol (BZAL), zearalanone (ZAN), tenuazonic acid (TEA), alternariol (AOH), alternariol mono-methyl-ether (AME), T2 toxin (T2).

### LC (Liquid Chromatography)

The next step of the method of the present invention is the qualitative and quantitative determination of the various mycotoxins and related phase I and II metabolites comprised in the blood sample.

This can be accomplished by subjecting the dried and reconstituted DBS sample to a separation by liquid chromatography. As set forth supra, such apparatus comprises a column comprising a stationary phase and a mobile (liquid) phase. In the column of such apparatus the mycotoxins and related metabolites are separated on the basis of their different affinity to on the one hand the stationary (or fixed) phase of the column, and on the other hand the mobile or liquid phase of such column. Given such differences in affinity, the residence time and hence the transfer time of the various mycotoxins and related metabolites through the column of the apparatus will be different, in term resulting in an appropriate separation of the individual mycotoxins.

A column suitable for use in the method of the present invention comprises HSS (High Strength Silica) Technology particles available from Waters ^{®} and its Benelux dealer, LabMakelaar Benelux BV Knibbelweg 18 C, Zevenhuizen, Nederland, e.g. the 1.8 µm High Strength Silica particle.

According to a preferred embodiment of the present invention, the composition of the liquid phase passing through the column of the liquid chromatography apparatus, is varied over time, according to the time of injection of the liquid phase into the column.

Tables 2 and 3 set forth supra illustrate the flow rate of the mobile phase as well as the gradient program applied.

The UPLC conditions for analytes determined using the ESI negative mode are as follows (see table 3):
at the start of the operation, the mobile phase comprises predominantly water (e.g. up to 95 %) and a small proportion of acetonitrile (e.g. up to 5 %), and as time goes by, the composition of the liquid phase is changed, whereby the amount of water gradually decreases (e.g. to approximately 40 %) and the amount of acetonitrile gradually increases (e.g. up to approximately 60 %).

Hereafter, the proportion of water again is increased substantially, up to 95 %, so as to recondition the column for future use.

The UPLC conditions for analytes determined using the ESI positive mode are as follows (see table 2):
at the start of the operation, the mobile phase comprises predominantly water (e.g. up to 95 %) and a small proportion of methanol (e.g. up to 5 %), and as time goes by, the composition of the liquid phase is changed, whereby the amount of water gradually decreases (e.g. to approximately 1 %) and the amount of methanol gradually increases (e.g. up to approximately 99 %).

Hereafter, the proportion of water again is increased substantially, up to 95 %, so as to recondition the column for future use.

As a result of the above, the various individual mycotoxins and related metabolites will exit the liquid chromatography column one after the other; at such stage, these compounds are suitable for being detected in the subsequent stage of the present invention, by the MS/MS apparatus.

Tables 4 and 5 set forth supra, illustrate the various retention times for the components comprised in the DBS sample to be analyzed.

### Experimental support:

Four different reversed phase columns (Hypersil Gold 50 mm × 2.1 mm, dp: 1.9, Thermo Scientific, Breda, The Netherlands; Zorbax Eclipse C18 50 mm × 2.1 mm, dp: 1.8, Agilent, Sint-Katelijne-Waver, Belgium; Acquity BEH-C18 50 mm × 2.1 mm, dp: 1.7, Waters, Milford, MA, USA; and Acquity HSS-T3 100 mm × 2.1 mm, dp: 1.8, Waters, Milford, MA, USA) were tested to achieve chromatographic separation of the selected mycotoxins. The best separation of all components was obtained on the HSS-T3 column.

The abbreviation HSS stands for High Strength Silica, the term T3 stands for a trifunctional C₁₈ alkyl chain.

The latter column is available as Acquity UPLC HSS T3 column from Waters Corporation, 34 Maple Street, Milford, USA.

For further details regarding the chromatography apparatus suitable for use in the method of the present invention, reference is made to reference document (3) as identified supra, paragraph 4.5, page 23 of 30.

### Detection of mycotoxins by MS-MS

For the detection of the mycotoxins separated by the LC apparatus, the MS-MS method and apparatus are used.

When this technique is used, the detection is performed on the basic ('precursor') molecule in a first stage, as well as on some fragments of such molecule in a second stage.

For further details regarding the MS-MS apparatus suitable for use in the method of the present invention, reference is made to reference document (3) as identified supra, paragraph 4.6.1, page 23 of 30.

### Method validation:

Upon completion development method according to the present invention, the inventors have undertaken the task of validating the method according to the present invention with respect to the DBS from pigs and broiler chickens.

For the purpose of validation, calibration curves have been set up, on the one hand with respect to the DBS from pigs, on the other hand with respect to the DBS from broiler chickens, in both cases for each of the mycotoxins and their phase I metabolites as set forth above.

To that end, tests have been run on three different days as well within a day, and the mean values have been calculated based on these results.

The results of these tests are described hereinafter.

Reference is hereby made to the following document, paragraph 2.2, page 5 of the Article entitled "Assessment of dried blood spots for multi-mycotoxin biomarker analysis in pigs and broiler chickens" by Marianne Lauwers, Siska Croubels, Siegrid de Baere, Milena Sevastiyanova, Eva Maria Romero Sierra, Ben Letor, Christos Gougoulias and Mathias Devreese, published on September 18, 2019 by Toxins 2019, 11, 541: doi:10.3390/toxins 11090541, www.mdpi.com/journal/toxins.

The linearity expressed as a correlation coefficient (r) and the goodness-of-fit (g) is shown in Table 1 for pig DBSs and Table 2 for chicken DBSs and is the mean ± standard deviation of three curves across three different days of analysis. The *r* ranged between 0.991 and 0.999 for pigs and between 0.993 and 0.998 for broiler chickens. The g-value varied from 6% to 17% in pig and 8% to 19% in broiler chicken DBSs. Most of the calibration curves matched a linear model with a 1/x weighing factor, except for the ENNs and BEA which are best described by a quadratic model with a 1/x weighing factor. For most mycotoxins, the linearity ranged between 0.5 and 200 ng·ml⁻¹. However, the limit of quantification (LOQ) and thus the lowest concentration in the calibration curve was set at 1 ng·ml⁻¹ for ZEN, AZAL, BZEL, ZAN, DOM1 and AME in pigs and for ZEN, AZAL, BZAL, ZAN, AOH, T2, ENNA and FB1 in broiler chickens. In broiler chickens, AZEL had an LOQ of 4 ng·ml⁻¹ and FB2 2 ng·ml⁻¹ and in pigs AOH had an LOQ of 10 ng·ml⁻¹. The results for the LOQ and corresponding LOD values are found in Table 1 and Table 3, respectively.

The within-day and between-day precision and accuracy at a concentration of 10 and 100 ng·ml⁻¹ met the requirements for all mycotoxins for both species. The results of the within-day and between-day precision and accuracy experiments can be found in the Tables set forth hereinafter, respectively.

The results for signal suppression or enhancement (SSE) after the extraction of DBSs from broiler chickens and pigs showed acceptable results (range 60-112%) for most components. Moreover, in pig DBSs, good extraction recovery rates were also observed. For some components, SSE was more pronounced and extraction recovery was rather low (<60%). However, for all mycotoxins, an adequate internal standard (IS) and matrix-matched calibration curves were used, resulting in validation results for accuracy and precision matching the acceptance criteria. The results for matrix effects and extraction recovery are shown in the Tables set forth is said publication.

For the sake of completeness, we reproduce hereinafter the table regarding the test results for pig whole blood:

**Table**

| Validation results for linearity shown as the mean ± standard deviation of three curves across three different days of analysis (linear range, correlation coefficient (r) and goodness-of-fit coefficient (g)), limit of quantification (LOQ) and limit of detection (LOD) of 23 mycotoxins in dried blood spots of pig whole blood. | | | | |
|---|---|---|---|---|
| **Analyte** | **Linearity (n = 3 Different Days)** | | **LOQ** | **LOD** |
| | **Linear Range** | **r ± SD** | | |
| | **(ng·ml-1)** | | **ng·mL⁻¹** | **ng·mL⁻¹** |
| ZEN | 1-200 | 0.999 ± 0.001 | 1.0 | 0.09 |
| AZEL | 0.5-200 | 0.998 ± 0.000 | 0.5 | 0.20 |
| AZAL | 1-200 | 0.998 ± 0.001 | 1.0 | 0.38 |
| BZAL | 0.5-200 | 0.997 ± 0.001 | 0.5 | 0.11 |
| BZEL | 1-200 | 0.994 ± 0.004 | 1.0 | 0.21 |
| ZAN | 1-200 | 0.996 ± 0.004 | 1.0 | 0.10 |
| TEA | 0.5-200 | 0.996 ± 0.003 | 0.5 | 0.04 |
| AOH | 10-200 | 0.994 ± 0.005 | 1.0 | 0.74 |
| AME | 1-200 | 0.993 ± 0.002 | 1.0 | 0.01 |
| DON | 0.5-200 | 0.994 ± 0.003 | 0.5 | 0.11 |
| DOM1 | 1-200 | 0.997 ± 0.002 | 1.0 | 0.23 |
| 3/15ADON | 0.5-200 | 0.994 ± 0.002 | 0.5 | 0.06 |
| T2 | 0.5-200 | 0.996 ± 0.004 | 0.5 | 0.01 |
| AFB1 | 0.5-200 | 0.994 ± 0.002 | 0.5 | 0.001 |
| AFM1 | 0.5-200 | 0.991 ± 0.002 | 0.5 | 0.01 |
| OTA | 0.5-200 | 0.995 ± 0.003 | 0.5 | 0.01 |
| ENN A1 | 0.5-200 | 0.997 ± 0.002 | 0.5 | 0.05 |
| ENNA | 0.5-100 | 0.998 ± 0.001 | 0.5 | 0.01 |
| ENNB | 0.5-100 | 0.999 ± 0.000 | 0.5 | 0.001 |
| ENNB1 | 0.5-100 | 0.998 ± 0.001 | 0.5 | 0.001 |
| BEA | 0.5-200 | 0.997 ± 0.003 | 0.5 | 0.02 |
| FB2 | 0.5-200 | 0.996 ± 0.002 | 0.5 | 0.35 |
| FB1 | 1-200 | 0.994 ± 0.004 | 0.5 | 0.23 |

For the sake of completeness, we reproduce hereinafter the table regarding the test results for broiler chicken whole blood:

**Table**

| Validation results for linearity shown as the mean ± standard deviation of three curves across three different days of analysis (linear range, correlation coefficient (r) and goodness-of-fit coefficient (g)), limit of quantification (LOQ) and limit of detection (LOD) of 23 mycotoxins in dried blood spots of broiler chicken whole blood. | | | | | |
|---|---|---|---|---|---|
| **Analyte** | **Linearity (n = 3 Different Days)** | | | **LOQ** | **LOD** |
| | **Linear Range** | **r ± SD** | **g ± SD** | | |
| | **(ng·ml-1)** | | | **ng·mL⁻¹** | **ng·mL⁻¹** |
| ZEN | 1-200 | 0.997 ± 0.001 17 ± 3 | | 1.0 | 0.12 |
| AZEL | 4-200 | 0.997 ± 0.002 8 ± 3 | | 4.0 | 1.10 |
| AZAL | 1-200 | 0.997 ± 0.002 16 ± 6 | | 1.0 | 0.15 |
| BZAL | 1-200 | 0.996 ± 0.003 18 ± 2 | | 1.0 | 0.27 |
| BZEL | 0.5-200 | 0.996 ± 0.003 16 ± 2 | | 0.5 | 0.1 |
| ZAN | 1-200 | 0.997 ± 0.001 13 ± 5 | | 1.0 | 0.21 |
| TEA | 0.5-200 | 0.998 ± 0.001 12 ± 6 | | 0.5 | 0.001 |
| AOH | 1-200 | 0.996 ± 0.002 17 ± 3 | | 1.0 | 0.02 |
| AME | 0.5-200 | 0.997 ± 0.001 17 ± 4 | | 0.5 | .001 |
| DON | 0.5-200 | 0.997 ± 0.000 15 ± 1 | | 0.5 | 0.18 |
| DOM1 | 0.5-200 | 0.998 ± 0.001 17 ± 1 | | 0.5 | 0.16 |
| 3/15ADON | 0.5-200 | 0.995 ± 0.003 16 ± 4 | | 0.5 | 0.09 |
| T2 | 1-200 | 0.998 ± 0.000 13 ± 5 | | 1.0 | 0.03 |
| AFB1 | 0.5-200 | 0.997 ± 0.001 15 ± 4 | | 0.5 | 0.01 |
| AFM1 | 0.5-200 | 0.997 ± 0.001 15 ± 2 | | 0.5 | 0.01 |
| OTA | 0.5-200 | 0.998 ± 0.001 19 ± 1 | | 0.5 | 0.05 |
| ENN A1 | 0.5-200 | 0.993 ± 0.002 14 ± 5 | | 0.5 | 0.04 |
| ENNA | 1-200 | 0.995 ± 0.003 16 ± 2 | | 1.0 | 0.11 |
| ENNB | 0.5-200 | 0.994 ± 0.001 12 ± 1 | | 0.5 | 0.07 |
| ENNB1 | 0.5-200 | 0.998 ± 0.002 12 ± 3 | | 0.5 | 0.001 |
| BEA | 0.5-200 | 0.996 ± 0.000 15 ± 4 | | 0.5 | 0.07 |
| FB2 | 1-200 | 0.997 ± 0.001 16 ± 3 | | 1.0 | 0.96 |
| FB1 | 2-200 | 0.995 ± 0.001 15 ± 3 | | 2.0 | 1.87 |

The present inventors have complemented the above results with data regarding linearity, limit of quantification (LOQ) and limit of detection (LOD) that were evaluated for the analytes that were added to the LC-MS/MS method as disclosed in this reference (2) and that were quantitatively determined, i.e. AFB2, AFG1 and AFG2.

These results are set forth in the table 7 below:

| | **Pig** | | |
|---|---|---|---|
| **Analyte** | Linear range (ng/ml) | LOQ (ng/ml) | LOD (ng/ml) |
| AFB2 | 0.5-200 | 0.5 | 0.02 |
| AFG1 | 0.5-200 | 0.5 | 0.03 |
| AFG2 | 0.5-200 | 0.5 | 0.04 |

| | | **Chicken** | |
|---|---|---|---|
| **Analyte** | Linear range (ng/ml) | LOQ (ng/ml) | LOD (ng/ml) |
| AFB2 | 0.5-200 | 0.5 | 0.03 |
| AFG1 | 0.5-200 | 0.5 | 0.02 |
| AFG2 | 0.5-200 | 0.5 | 0.05 |

### Real farming conditions

Field DBS samples originating from chickens and pigs coming from the real farming production, where animals are encountering naturally contaminated feed, were analyzed using the updated LC-MS/MS method. This is a difference compared to the article described by Lauwers et al (reference article 3] were only DBS samples originating of a toxicokinetic study with DON, ZEN and AFB1 in pigs and OTA, AFB1 and DON in broiler chickens were analyzed.

The results of this multi-mycotoxin analysis in a real chicken and pig DBS sample demonstrate the applicability updated LC-MS/MS method analysis in ESI positive and negative mode, respectively.

### Advantages of the present invention:

The advantages of the method according to the present invention:
As compared to monitoring the animal feed, the present invention offers the following advantages:
1) Biomonitoring measures the exposure at an individual level while feed analysis shows a general risk. Exposure at an individual level takes variation in food consumption and ADME parameters into account and integrates contamination from all different sources leading to a more accurate estimation and possible use in toxicokinetic studies.
   (ADME stands for Absorption, distribution, metabolism, excretion, all of these toxicokinetic parameters describing the effect of the animal body on the toxins concerned,);
2) Although feed is an accessible matrix, it is difficult to sample correctly due to the presence of hotspots. Urine, feces and in particular plasma/blood in contrast are easy to sample correctly and also allow sequential sampling;
3) Measuring complete exposure: modified mycotoxins can convert back to their original forms during digestion and so contribute to the overall effect of the toxins. Biomonitoring includes these effects and gives thus a very accurate estimation of the risk;
4) Use of one LC-MS/MS apparatus is sufficient compared to the prior art method, requiring the additional use of an LC-HRMS equipment;
5) Broad screening or multi-mycotoxin biomonitoring directed to 36 components compared to the prior-art method, limited to 24 components.

### Biomonitoring analysis as a new tool to evaluate mycotoxin detoxifiers:

A common way to counter the harmful effect of mycotoxins comprises adding mycotoxins detoxifiers to the animal feed.

An example of such detoxifier is the product marketed by Innovad N.V., Cogels-Osylei 33, 2600 Antwerp, Belgium, under the brand name Escent S ^{®}.

A detoxifier product is a product that diminishes the effects associated with toxins. A possible way to prove the efficacy of these products is doing *in vivo* absorption tests. In these trials the concentration of mycotoxins in animal matrices is detected and the difference in concentration with and without detoxifier is determined. This difference is a measure for the efficacy of the product. This type of test gives accurate results and should in the future replace *in vitro* tests or *in vivo* test based on non-specific parameters such as growth performance and feed conversion.

## Claims

1. Method for the detection of the following (10) mycotoxins and metabolites in broiler chickens or pigs,
- Aflatoxin B1 (AFB1),
- Aflatoxin B2 (AFB2),
- Aflatoxin M1 (AFM1),
- Fumonisin (FB1),
- Fumonisin (FB2),
- Ochratoxin A (OTA),
- Deoxynivalenol (DON),
- T2 toxin (T2),
- HT-2-toxin (HT2),
- Zearalenone (ZEN),
said method comprising:
- collecting the blood of broiler chickens or pigs as a dried blood sample;
- preparing the dried blood sample for analysis;
- analyzing the prepared dried blood sample by liquid chromatography-tandem mass spectrometry (LC-MS/MS) in two steps:
a) detecting in a LC-MS/MS step,
- the reversed phase LC process using as mobile phase, a mixture of acetic acid, acetonitrile and water, the proportion of the acetonitrile solution over the water solution gradually increasing during the process, and at the end diminishing to recondition the LC column,
- the mass spectrometer operating in negative electro-spray ionization mode,
and
b) detecting in another LC-MS/MS step,
- the reversed phase LC process using as mobile phase, a mixture of ammonium formate, formic acid, water and methanol, the proportion of the methanol solution over the water solution gradually increasing during the process, and at the end diminishing to recondition the LC column,
- the mass spectrometer operating in positive electro-spray ionization mode.

2. Method according to claim 1 for the detection of the following (8) additional mycotoxins and metabolites:
- Alternariol methyl ether (AME),
- Alternariol (AOH),
- Tenuazonic Acid (TEA),
- Beauvericin (BEA),
- Enniatin A, A1, B, B1 (ENNA, ENNA1, ENNB, ENNB1).

3. Method according to claim 2 for the detection of the following (18) additional mycotoxins and metabolites:
- de-epoxy-deoxynivalenol (DOM1),
- 15-acetyldeoxynivalenol (15ADON),
- 3-acetyldeoxynivalenol (3ADON),
- Zearalanone (ZAN),
- α-Zearalenol (AZEL),
- α-Zearalanol (AZAL),
- β-Zearalanol (BZAL),
- β-Zearalenol (BZEL),
- DON-glucuronide (DON-GlcA),
- DON-Sulphate (DON-S),
- ZEN-glucuronide (ZEN-GlcA),
- ZEN-sulfate (ZEN-S),
- α-zearalenol-glucuronide (A-ZEL-GlcA),
- β-zearalenol-glucuronide (B-ZEL-GlcA),
- Fumonisin B3 (FB3),
- Aflatoxin M2 (AFM2),
- Aflatoxin G 1 (AFG1),
- Aflatoxin G2 (AFG2).

4. Method according to any of the preceding claims wherein any of the following (15) mycotoxins and metabolites are detected by the mass spectrometer operating in negative electrospray ionization mode:
- zearalenone (ZEN),
- zearalanone (ZAN),
- α-zearalenol (AZEL)
- α-zearalanol (AZAL),
- β-zearalanol (BZAL),
- β-zearalenol (BZEL),
- tenuazonic acid (TEA),
- alternariol (AOH),
- alternariol methyl ether (AME),
- deoxynivalenol sulphate (DON-S),
- deoxynivalenol glucuronide (DON-GlcA),
- zearalenone sulphate (ZEN-S),
- zearalenone glucuronide (ZEN-GlcA),
- a-ZEL-glucuronide (AZEL-GlcA),
- b-ZEL- glucuronide (BZEL-GlcA).

5. Method according to any of the preceding claims wherein any of the following (21) mycotoxins and metabolites are detected by the mass spectrometer operating in positive electrospray ionization mode:
- deoxynivalenol (DON),
- de-epoxy-deoxynivalenol (DOM1),
- 3-acetyldeoxynivalenol (3ADON),
- 15-acetyldeoxynivalenol (15ADON),
- T2-toxin (T2),
- HT-2 toxin (HT2),
- aflatoxin B1 (AFB1),
- aflatoxin M1 (AFM1),
- ochratoxin A (OTA),
- enniatin A1 (ENNA1),
- enniatin A (ENNA),
- enniatin B (ENNB),
- enniatin B1(ENNB1),
- beauvericin (BEA),
- fumonisin (FB1),
- fumonisin (FB2),
- aflatoxin B2 (AFB2),
- aflatoxin G1, (AFG1),
- aflatoxin G2 (AFG2),
- aflatoxin M2, (AFM2)
- fumonisin B3 (FB3).

6. Method according to any of the preceding claims, whereby
- in the LC-MS/MS step, using as mobile phase, a mixture of an acetic acid, acetonitrile and water solution, the overall process time amounts to 11 min or less, wherein the proportion of the acetonitrile solution over the water solution gradually increases from 5/95 % to 60/40 % in a time period of maximum 8 min, thereafter decreases to 5/95 % for a 3 minutes time period to recondition the LC column,
and/or,
- in the LC-MS/MS step, using as mobile phase, a mixture of ammonium formate, formic acid, water and methanol, the overall process time amounts 12 min or less, wherein the proportion of the methanol solution over the water solution gradually increases from 5/95 % to 99/1 % in a time period of maximum 9 min, thereafter decreases to 5/95 % for a 3 minutes time period to recondition the LC column.

7. Method according to any of the preceding claims, wherein in the LC process a column is used comprising an hydrophobic stationary phase comprising silica with covalently bonded alkyl chains, preferably high strength silica with trifunctional C₁₈ alkyl bonded chains.

8. Method according to any of the preceding claims, comprising, prior to analyzing, extracting the mycotoxins and the metabolites from the dried blood sample in an extraction solvent and subjecting the extraction solvent and the dried blood sample to an ultrasonic bath treatment.

9. Method according to claim 8, whereby the extraction solvent comprises a water/acetonitrile/acetone mixture.

10. Method according to claim 8, comprising drying the extraction solvent and reconstituting the dried mass in a reconstitution solvent.

11. Method according to claim 10, whereby the reconstituting solvent comprises a water/methanol/formic acid mixture.

12. Method according to claim 1, further comprising spiking the collected dried blood sample with one or more internal standards.

13. Method according to claim 12, comprising spiking the collected dried blood sample with one or more internal standards selected from the following list:
- ¹³C₁₅-deoxynivalenol,
- ¹³C₁₇-aflatoxin B1,
- ¹³C₁₇-aflatoxin M1,
- ¹³C₂₀-ochratoxin A,
- ¹³C₂₄-T2-toxin,
- ¹³C₃₄-fumonisin B1,
- ¹⁵N₃-enniatin B,
- ¹³C₆¹⁵N-tenuazonic acid,
- ¹³C₁₈-zearalenone.

14. Use of the method of any of the preceding claims for screening and assessing the exposure of pigs or broiler chickens to feed contaminated with mycotoxins.

15. Use of the method of any of the preceding claims for assessing the impact of the addition of mycotoxin detoxifying agents to animal feed.

## Patentansprüche

1. Methode zum Nachweis der folgenden (10) Mykotoxine und Metaboliten in Broilerhühnern oder Schweinen,
- Aflatoxin B1 (AFB1),
- Aflatoxin B2 (AFB2),
- Aflatoxin M1 (AFM1),
- Fumonisin (FB1),
- Fumonisin (FB2),
- Ochratoxin A (OTA),
- Deoxynivalenol (DON),
- T2-Toxin (T2),
- HT-2-Toxin (HT2),
- Zearalenon (ZEN),
wobei das Verfahren aufweist:
- Entnehmen des Blutes von Masthühnern oder Schweinen in Form einer getrockneten Blutprobe;
- Aufbereiten der getrockneten Blutprobe für eine Analyse;
- Analysieren der aufbereiteten getrockneten Blutprobe durch Flüssigchromatographie-Tandem-Massenspektrometrie (LC-MS/MS) in zwei Schritten:
a) Nachweisen in einem LC-MS/MS-Schritt,
- das Umkehrphasen-LC-Verfahren verwendet als mobile Phase ein Gemisch aus Essigsäure, Acetonitril und Wasser, wobei der Anteil der Acetonitril-Lösung gegenüber der Wasserlösung während des Prozesses allmählich zunimmt und am Ende abnimmt, um die LC-Säule zu rekonditionieren,
- das Massenspektrometer arbeitet im negativen Elektrospray-Ionisierungsmodus,
und
b) Nachweisen in einem weiteren LC-MS/MS-Schritt,
- das Umkehrphasen-LC-Verfahren verwendet als mobile Phase eine Mischung aus Ammoniumformiat, Ameisensäure, Wasser und Methanol, wobei der Anteil der Methanollösung gegenüber der Wasserlösung während des Prozesses allmählich zunimmt und am Ende abnimmt, um die LC-Säule zu rekonditionieren,
- das Massenspektrometer arbeitet im positiven Elektrospray-Ionisierungsmodus.

2. Verfahren nach Anspruch 1 zum Nachweis der folgenden (8) zusätzlichen Mykotoxine und Metaboliten:
- Alternariol-Methylether (AME),
- Alternariol (AOH),
- Tenuazonsäure (TEA),
- Beauvericin (BEA),
- Enniatin A, A1, B, B1 (ENNA, ENNA1, ENNB, ENNB1).

3. Verfahren nach Anspruch 2 zum Nachweis der folgenden (18) zusätzlichen Mykotoxine und Metaboliten:
- De-Epoxy-Deoxynivalenol (DOM1),
- 15-Acetyldeoxynivalenol (15ADON),
- 3-Acetyldeoxynivalenol (3ADON),
- Zearalanon (ZAN),
- α-Zearalenol (AZEL),
- α-Zearalanol (AZAL),
- ß-Zearalanol (BZAL),
- ß-Zearalenol (BZEL),
- DON-Glucuronid (DON-GlcA),
- DON-Sulfat (DON-S),
- ZEN-Glucuronid (ZEN-GlcA),
- ZEN-Sulfat (ZEN-S),
- α-Zearalenol-Glucuronid (A-ZEL-GlcA),
- ß-Zearalenol-Glucuronid (B-ZEL-GlcA),
- Fumonisin B3 (FB3),
- Aflatoxin M2 (AFM2),
- Aflatoxin G1 (AFG1),
- Aflatoxin G2 (AFG2).

4. Verfahren nach einem der vorhergehenden Ansprüche, wobei eines der folgenden (15) Mykotoxine und Metaboliten durch das Massenspektrometer, das im negativen Elektrospraylonisationsmodus arbeitet, nachgewiesen wird:
- Zearalenon (ZEN),
- Zearalanon (ZAN),
- α-Zearalenol (AZEL)
- α-Zearalanol (AZAL),
- ß-Zearalanol (BZAL),
- ß-Zearalenol (BZEL),
- Tenuazonsäure (TEA),
- Alternariol (AOH),
- Alternariol-Methylether (AME),
- Deoxynivalenol-Sulfat (DON-S),
- Deoxynivalenol-Glucuronid (DON-GlcA),
- Zearalenon-Sulfat (ZEN-S),
- Zearalenon-Glucuronid (ZEN-GlcA),
- a-ZEL-Glucuronid (AZEL-GlcA),
- b-ZEL-Glucuronid (BZEL-GlcA).

5. Verfahren nach einem der vorhergehenden Ansprüche, wobei eines der folgenden (21) Mykotoxine und Metaboliten durch das Massenspektrometer, das im positiven Elektrospraylonisationsmodus arbeitet, nachgewiesen wird:
- Deoxynivalenol (DON),
- De-Epoxy-Deoxynivalenol (DOM1),
- 3-Acetyldeoxynivalenol (3ADON),
- 15-Acetyldeoxynivalenol (15ADON),
- T2-Toxin (T2),
- HT-2-Toxin (HT2),
- Aflatoxin B1 (AFB1),
- Aflatoxin M1 (AFM1),
- Ochratoxin A (OTA),
- Enniatin A1 (ENNA1),
- Enniatin A (ENNA),
- Enniatin B (ENNB),
- Enniatin B1 (ENNB1),
- Beauvericin (BEA),
- Fumonisin (FB1),
- Fumonisin (FB2),
- Aflatoxin B2 (AFB2),
- Aflatoxin G1, (AFG1),
- Aflatoxin G2 (AFG2),
- Aflatoxin M2, (AFM2)
- Fumonisin B3 (FB3).

6. Verfahren nach einem der vorhergehenden Ansprüche, wobei
- im LC-MS/MS-Schritt, bei dem als mobile Phase ein Gemisch aus einer Essigsäure, Acetonitril und Wasser als mobile Phase verwendet wird, die Gesamtprozesszeit 11 min oder weniger beträgt, wobei der Anteil der Acetonitrillösung gegenüber der Wasserlösung allmählich von 5/95 % auf 60/40 % in einer Zeitspanne von maximal 8 min ansteigt, danach für einen Zeitraum von 3 Minuten auf 5/95 % abfällt, um die LC-Säule zu rekonditionieren,
und/oder,
- im LC-MS/MS-Schritt, bei dem als mobile Phase ein Gemisch aus Ammoniumformiat, Ameisensäure, Wasser und Methanol verwendet wird, die Gesamtprozesszeit 12 min oder weniger beträgt, wobei der Anteil der Methanollösung gegenüber der Wasserlösung schrittweise von 5/95 % auf 99/1 % in einer Zeitspanne von maximal 9 min ansteigt, danach für eine Zeitspanne von 3 Minuten auf 5/95 % abfällt, um die LC-Säule zu rekonditionieren.

7. Verfahren nach einem der vorhergehenden Ansprüche, wobei in dem LC-Verfahren eine Säule verwendet wird, die eine hydrophobe stationäre Phase aufweist, die Siliciumdioxid mit kovalent gebundenen Alkylketten, vorzugsweise hochfeste Kieselsäure mit trifunktionalen C₁₈-Alkyl-gebundenen Ketten, aufweist.

8. Verfahren nach einem der vorhergehenden Ansprüche, wobei vor der Analyse die Mykotoxine und die Metaboliten aus der getrockneten Blutprobe in ein Extraktionslösungsmittel extrahiert werden und das Extraktionslösungsmittel und die getrocknete Blutprobe einer Ultraschallbadbehandlung unterzogen werden.

9. Verfahren nach Anspruch 8, wobei das Extraktionslösungsmittel ein Wasser/Acetonitril/Aceton-Gemisch aufweist.

10. Verfahren nach Anspruch 8, aufweisend Trocknen des Extraktionslösungsmittels und Rekonstituieren der getrockneten Masse in einem Rekonstitutionslösungsmittel.

11. Verfahren nach Anspruch 10, wobei das Rekonstitutionslösungsmittel ein Wasser/Methanol/Ameisensäure-Gemisch aufweist.

12. Verfahren nach Anspruch 1, das ferner ein Hinzufügen eines oder mehrerer interner Standards zur gesammelten getrockneten Blutprobe aufweist.

13. Verfahren nach Anspruch 12, weiter aufweisend ein Hinzufügen eines oder mehrerer interner Standards, ausgewählt aus der folgenden Liste, zur gesammelten getrockneten Blutprobe:
- ¹³C₁₅-Desoxynivalenol,
- ¹³C₁₇-Aflatoxin B1,
- ¹³C₁₇-Aflatoxin M1,
- ¹³C₂₀-Ochratoxin A,
- ¹³C₂₄-T2-Toxin,
- ¹³C₃₄-Fumonisin B1,
- ¹⁵N₃-Neniatin B,
- ¹³C₆¹⁵N-Tenuazonsäure,
- ¹³C₁₈-Zearalenon.

14. Verwendung des Verfahrens nach einem der vorhergehenden Ansprüche zum Screening und zur Bewertung der Exposition von Schweinen oder Masthühnern gegenüber mit Mykotoxinen kontaminiertem Futter.

15. Verwendung des Verfahrens nach einem der vorangehenden Ansprüche zur Bewertung der Auswirkungen des Zusatzes von Mykotoxin-Entgiftungsmitteln zu Tierfutter.

## Revendications

1. Méthode pour la détection des (10) mycotoxines et métabolites suivantes dans les poulets d'engraissement et les cochons,
- Aflatoxine B1 (AFB1),
- Aflatoxine B2 (AFB2),
- Aflatoxine M1 (AFM1),
- Fumonisine (FB1),
- Fumonisine (FB2),
- Ochratoxine A (OTA),
- Deoxynivalenol (DON),
- T2-Toxine (T2),
- HT-2-Toxine (HT2),
- Zearalenone (ZEN),
ladite méthode comprenant :
- la collection du sang de poulets d'engraissement ou de cochons en tant qu'échantillon de sang séché;
- la préparation de l'échantillon de sang séché pour analyse;
- l'analyse de l'échantillon du sang séché préparé par chromatographie liquide spectrométrie de masse tandem (LC-MS/MS) dans deux étapes:
a) la détection dans une étape LC-MS/MS,
- le procédé LC en phase inversée, utilisant en tant que phase mobile un mélange d'acide acétique, d'acétonitrile et d'eau, la proportion de la solution d'acétonitrile par rapport à la solution aqueuse augmentant graduellement durant le procédé, et à la fin diminuant pour reconditionner la colonne LC,
- le spectromètre de masse opérant dans un mode d'ionisation par électro-pulvérisation négative,
et
b) la détection dans une autre étape LC-MS/MS,
- le procédé LC en phase inversée utilisant en tant que phase mobile, un mélange d' ammonium formate, d'acide formique, de l'eau et du méthanol, la proportion de la solution de méthanol par rapport à la solution aqueuse augmentant graduellement durant le procédé, et à la fin diminuant pour reconditionner la colonne LC,
- le spectromètre de masse opérant dans un mode d'ionisation par électro-pulvérisation positive.

2. Méthode selon la revendication 1 pour la détection des (8) mycotoxines et métabolites supplémentaires suivants :
- Alternariole-Methylether (AME),
- Alternariole (AOH),
- Acide Tenuazonique (TEA),
- Beauvericine (BEA),
- Enniatine A, A1, B, B1 (ENNA, ENNA1, ENNB, ENNB1).

3. Méthode selon la revendication 2 pour la détection des (18) mycotoxines et métabolites supplémentaires suivants :
- de-epoxy-deoxynivalenole (DOM1),
- 15-acetyldeoxynivalenole (15ADON),
- 3-Acetyldeoxynivalenole (3ADON),
- Zearalanone (ZAN),
- α-Zearalenole (AZEL),
- α-Zearalanole (AZAL),
- ß-Zearalanole (BZAL),
- ß-Zearalenole(BZEL),
- DON-Glucuronide (DON-GlcA),
- DON-Sulfate (DON-S),
- ZEN-Glucuronide (ZEN-GlcA),
- ZEN-Sulfate (ZEN-S),
- a-Zearalenol-Glucuronide (A-ZEL-GlcA),
- ß-Zearalenol-Glucuronide(B-ZEL-GlcA),
- Fumonisine B3 (FB3),
- Aflatoxine M2 (AFM2),
- Aflatoxine G1 (AFG1),
- Aflatoxine G2 (AFG2).

4. Méthode selon l'une quelconque des revendications précédentes, dans laquelle n'importe quelle des (15) mycotoxines et métabolites suivants sont détectés par le spectromètre de masse opérant dans un mode d'ionisation par électro-pulvérisation négative :
- Zearalenone (ZEN),
- Zearalanone (ZAN),
- α-Zearalenole (AZEL)
- α-Zearalanole (AZAL),
- ß-Zearalanole (BZAL),
- ß-Zearalenole (BZEL),
- AcideTenuazone (TEA),
- Alternariole (AOH),
- Alternariole-Methylether (AME),
- Sulfate de Deoxynivalenol (DON-S),
- Deoxynivalenol-Glucuronide (DON-GlcA),
- Sulfate de Zearalenone (ZEN-S),
- Zearalenone-Glucuronide (ZEN-GlcA),
- α-ZEL-Glucuronide (AZEL-GlcA),
- b-ZEL-Glucuronide (BZEL-GlcA).

5. Méthode selon l'une quelconque des revendications précédentes, dans laquelle n'importe quelle des (21) mycotoxines et métabolites suivants sont détectés par le spectromètre de masse opérant dans un mode d'ionisation par électro-pulvérisation positive :
- Deoxynivalenole (DON),
- De-Epoxy-Deoxynivalenole (DOM1),
- 3-Acetyldeoxynivalenole (3ADON),
- 15-Acetyldeoxynivalenole (15ADON),
- T2-Toxine (T2),
- HT-2-Toxine (HT2),
- Aflatoxine B1 (AFB1),
- Aflatoxine M1 (AFM1),
- Ochratoxine A (OTA),
- Enniatine A1 (ENNA1),
- Enniatine A (ENNA),
- Enniatine B (ENNB),
- Enniatine B1 (ENNB1),
- Beauvericine (BEA),
- Fumonisine (FB1),
- Fumonisin e(FB2),
- Aflatoxine B2 (AFB2),
- Aflatoxine G1, (AFG1),
- Aflatoxine G2 (AFG2),
- Aflatoxine M2, (AFM2)
- Fumonisine B3 (FB3).

6. Méthode selon l'une quelconque des revendications précédentes, dans laquelle
- dans l'étape LC-MS/MS, utilisant pour la phase mobile un mélange d'acide acétique, d'acétonitrile et de solution aqueuse, le temps global du procédé comprend 11 minutes ou moins, dans laquelle la proportion de la solution d'acétonitrile par rapport à la solution aqueuse augmente graduellement de 5/95 % à 60/40% dans une période de temps de maximum 8 minutes, ensuite diminue vers 5/95% pour une période de temps de 3 minutes pour reconditionner la colonne LC,
et/ou,
- dans l'étape LC-MS/MS, utilisant pour la phase mobile un mélange d'ammonium formate, d'acide formique, d'eau et de méthanol, le temps global du procédé comprend 12 minutes ou moins, dans laquelle la proportion de la solution méthanol par rapport à la solution aqueuse augmente graduellement de 5/95 % à 99/1% dans une période de temps de maximum 9 minutes, ensuite diminue vers 5/95% pour une période de temps de 3 minutes pour reconditionner la colonne LC.

7. Méthode selon l'une quelconque des revendications précédentes selon laquelle dans le procédé LC une colonne est utilisée comprenant une phase stationnaire hydrophobique comprenant du silica avec des chaines alkyles liées de manière covalente, de préférence du silica à haute résistance avec des chaînes liées tri-fonctionellement C₁₈-Alkyle.

8. Méthode selon l'une quelconque des revendications précédentes, comprenant, avant l'analyse, l'extraction des mycotoxines et des métabolites de l'échantillon de sang séché dans un solvant d'extraction et soumission du solvant d'extraction et de l'échantillon de sang séché à un traitement de bain ultrasonique.

9. Méthode selon l'une quelconque des revendications précédentes, dans laquelle le solvant d'extraction comprend un mélange d'eau/acétonitrile/acétone.

10. Méthode selon la revendication 8, comprenant le séchage du solvant d'extraction et la reconstitution de la masse séchée dans un solvant de reconstitution.

11. Méthode selon la revendication 10, dans laquelle le solvant de reconstitution comprend un mélange d'eau/méthanol/acide formique.

12. Méthode selon la revendication 1, comprenant en outre l'ajout à l'échantillon de sang séché collecté d'un ou plusieurs standards internes.

13. Méthode selon la revendication 12, comprenant l'ajout à l'échantillon de sang séché collecté d'un ou de plusieurs standards internes choisis parmi la liste suivante:
- ¹³C₁₅-Desoxynivalenole,
- ¹³C₁₇-Aflatoxine B1,
- ¹³C₁₇-Aflatoxine M1,
- ¹³C₂₀-Ochratoxine A,
- ¹³C₂₄-T2-Toxine,
- ¹³C₃₄-Fumonisin eB1,
- ¹⁵N₃-Neniatine B,
- ¹³C₆¹⁵N Acide Tenuazone,
- ¹³C₁₈-Zearalen.

14. Usage de la méthode selon l'une quelconques des revendications précédentes pour le dépistage et l'évaluation de l'exposition des cochons ou des poulets d'engraissement à une nourriture contaminée avec des mycotoxines.

15. Usage de la méthode selon l'une quelconques des revendications précédentes pour l'évaluation de l'effet de l'addition d'agents détoxifiants de mycotoxines à la nourriture animale.
